(19)

Europäisches Patentamt

European Patent Office

Office européen des brevets

(11) **EP 4 198 042 A1**

(12) **EUROPEAN PATENT APPLICATION**
published in accordance with Art. 153(4) EPC

(43) Date of publication:
**21.06.2023 Bulletin 2023/25**

(21) Application number: **21856228.8**

(22) Date of filing: **11.08.2021**

(51) International Patent Classification (IPC):
*C07K 14/00* $^{(2006.01)}$   *C07K 7/08* $^{(2006.01)}$
*G01N 33/574* $^{(2006.01)}$   *A61K 47/42* $^{(2017.01)}$
*A61K 31/704* $^{(2006.01)}$   *A61K 38/00* $^{(2006.01)}$
*A61K 51/08* $^{(2006.01)}$

(52) Cooperative Patent Classification (CPC):
**A61K 31/704; A61K 38/00; A61K 47/42;**
**A61K 51/08; C07K 7/08; C07K 14/00; G01N 33/574**

(86) International application number:
**PCT/KR2021/010657**

(87) International publication number:
**WO 2022/035222 (17.02.2022 Gazette 2022/07)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **11.08.2020   KR 20200100354**
**10.08.2021   KR 20210105103**

(71) Applicant: **Kyungpook National University**
**Industry-Academic**
**Cooperation Foundation**
**Daegu 41566 (KR)**

(72) Inventors:
• **LEE, Byung-Heon**
**Daegu 42038 (KR)**

• **CHAE, Youngji**
**Daegu 41245 (KR)**
• **KHAN, Fatima**
**Daegu 41944 (KR)**
• **VADEVOO, Sri Murugan Poongkavithai**
**Daegu 41907 (KR)**
• **GUNASSEKARAN, Gowri Rangaswamy**
**Daegu 41907 (KR)**

(74) Representative: **Roos, Peter**
**c/o Roospatent**
**Noldinstrasse 7**
**81545 München (DE)**

Remarks:
The complete document including Reference
Table(s) and the Sequence Listing(s) can be
downloaded from the EPO website

(54) **PEPTIDE SELECTIVELY BINDING TO CANCER CELL-DERIVED EXOSOME, AND USES THEREOF**

(57)    The present invention relates to a peptide selectively binding to a cancer cell-derived exosome, and uses thereof. A peptide of ExoPep (CRKVAKG) has been discovered using a phage display technique to discover a peptide with specific binding ability to an exosome derived from cancer cells. The peptide binds to a cancer cell-derived exosome having a property of moving to a cancer site and a cancer metastasis site, and thus may have the effect of delivering an anticancer drug to the cancer metastasis site.

EP 4 198 042 A1

[FIG. 14]

# Description

## TECHNICAL FIELD

The present disclosure relates to a peptide selectively binding to a cancer cell-derived exosome, and uses thereof.

## BACKGROUND ART

Exosomes are small vesicles secreted by cells and contain proteins, mRNAs, and miRNAs expressed by cells, representing characteristics of a cell by itself. Exosomes are involved in cell-cell communication by delivering the substance to other cells. Particularly, cancer cell-derived exosomes exist in high concentrations in the blood and are known to help the cancer metastasize by moving to a site where cancer metastasis takes place. Many studies have revealed that exosomes are very actively secreted from tumor cells and play a role in delivering substances necessary for cancer metastasis and development to the metastasis site.

Cancer is one of the most common diseases worldwide, and treatments that are currently applied include surgery, radiation, and chemotherapy. Although the molecular mechanisms of cancer are being actively studied, a majority of currently developed treatments depend on surgery. However, recently, various targeted therapeutic agents such as small molecule inhibitors, monoclonal antibody, and short targeting peptides targeting cancer cells have been developed and applied as therapeutic agents. In particular, short targeting peptides have high tissue permeability and are low in toxicity and immune response, thereby ensuring high potential as an effective anticancer agent.

Phage display of peptides and antibody is a very useful method of identifying ligands specific to target cells, and widely used to discover peptides and antibodies targeting cancer cells in vitro and in vivo. Accordingly, researchers of the present disclosure intended to discover peptides targeting exosomes by using the property that cancer cell-derived exosomes, which are drawing attention lately, move to a site of cancer development and a site of cancer metastasis while circulating in the blood, thereby applying the same to the delivery of anticancer drugs and anticancer treatment.

## DISCLOSURE

## TECHNICAL GOALS

The present disclosure relates to a peptide specifically binding to a cancer cell-derived exosome and uses thereof. Specifically, an object of the present disclosure is to provide a peptide specifically binding to a cancer cell-derived exosome having an amino acid sequence represented by SEQ ID NO: 1, a composition for diagnosing cancer, a composition for drug delivery, a composition for imaging cancer cells, and a composition for detecting cancer cell-derived exosomes including the peptide as an active ingredient, a fusion peptide in which an apoptosis-inducing peptide having an amino acid sequence represented by SEQ ID NO: 2 is bound to the peptide, a pharmaceutical composition for preventing or treating cancer including the fusion peptide as an active ingredient, and a pharmaceutical composition for preventing or treating cancer including the fusion peptide and an anticancer agent as active ingredients.

## TECHNICAL SOLUTIONS

To solve the above issues, example embodiments of the present disclosure provide a peptide specifically binding to a cancer cell-derived exosome, having an amino acid sequence represented by SEQ ID NO: 1.

In addition, example embodiments of the present disclosure provide a polynucleotide encoding the peptide, a recombinant vector including the polynucleotide, and a transformant transformed with the recombinant vector.

In addition, example embodiments of the present disclosure provide a composition for diagnosing cancer including the peptide as an active ingredient.

In addition, example embodiments of the present disclosure provide a composition for drug delivery including the peptide as an active ingredient.

In addition, example embodiments of the present disclosure provide a fusion peptide in which an apoptosis-inducing peptide having an amino acid sequence represented by SEQ ID NO: 2 is bound to the peptide, and a pharmaceutical composition for preventing or treating cancer including the fusion peptide as an active ingredient.

In addition, example embodiments of the present disclosure provide a pharmaceutical composition for preventing or treating cancer including the fusion peptide and an anticancer agent as active ingredients.

In addition, example embodiments of the present disclosure provide a composition for imaging cancer cells including the peptide as an active ingredient.

In addition, example embodiments of the present disclosure provide a composition for detecting cancer cell-derived exosomes including the peptide as an active ingredient.

## ADVANTAGEOUS EFFECTS

Example embodiments of the present disclosure relate to a peptide selectively binding to a cancer cell-derived exosome, and uses thereof. A peptide of ExoPep (CRKVAKG) has been discovered using a phage display technique to discover a peptide with binding ability specific to an exosome derived from cancer cells. The peptide binds to a cancer cell-derived exosome having a property of migrating to a cancer site and a cancer metastasis site, and thus may have the effect of delivering an anticancer drug to the cancer metastasis site.

BRIEF DESCRIPTIONS OF THE DRAWINGS

FIG. 1 shows results of analyzing characteristics of exosomes isolated from various cell lines using ultracentrifuge and ExoQuick reagent.

FIG. 2 shows a phage library screening process, fluctuations in phage titers, and sequences of selected candidate peptides for the discovery of tumor-derived exosome bound peptides.

FIG. 3 shows results of evaluating the binding ability of screened 10 phage clones to exosomes and exosome-producing cells through an ELISA assay.

FIG. 4 shows results of evaluating the binding ability of ExoPep peptides to exosomes derived from A549, MDA-MB231, MCF7, HEK293, and MCF10A cells and cells producing each exosome using a flow cytometer, and results of evaluating internalization of ExoPep peptides bound to exosomes into cells using a confocal microscope.

FIG. 5 shows results of investigating inhibition of exosomes internalized into cells using a confocal fluorescence microscopy by reacting A549 tumor cell-derived exosomes with ExoPep peptides labeled with magnetic particles and then eliminating the peptide-bound exosomes using a magnet.

FIG. 6 shows results of analyzing, by confocal microscopy, inhibition of exosome release of A549 cells by treatment of GW4869 which is an exosome production inhibitor, effects on cell survival, and fluctuation in internalization of ExoPep peptides thereby.

FIG. 7 shows results of analyzing in vitro binding of ExoPep peptides using a flow cytometer by isolating exosomes derived from A549 tumor mouse blood and normal mouse blood and results of analyzing internalization into cells with a confocal microscope.

FIG. 8 shows results of a western blotting analysis for the characteristics of exosomes isolated with streptavidin beads or CD63 antibody beads respectively after injection of biotin-ExoPep peptide into the blood of A549 tumor mice and normal mice, and results of hemolytic activity according to the concentration of ExoPep peptide for red blood cells.

FIG. 9 shows results of image and histological analysis of in vivo distribution of A549 tumor mouse blood-derived exosomes and A549 cell-derived exosomes with or without being bound to ExoPep peptide in A549 tumor mice.

FIG. 10 shows results of identifying cytotoxicity by reacting exosomes isolated from cancer cells (A549, MDA-MB231, Panc-1, HT29, HepG2) and normal HEK293 cells with ExoPep-KLA peptide, and then treating the same to each of cells producing each exosome.

FIG. 11 shows results of measuring induction of apoptosis by the phosphorescence intensity emitted from cells, by reacting exosomes isolated from A549 and MDA-MB231 cell lines with ExoPep-KLA peptide and then treating the same to each of the cells producing the exosomes.

FIG. 12 shows results of measuring A549 apoptosis by ExoPep-KLA peptide bound to A549 cell-derived exosomes and A549 tumor mouse blood-derived exosomes in a percentage of cells stained with annexin V.

FIG. 13 shows results of analyzing the stability of ExoPep-KLA peptide in serum.

FIG. 14 shows results of analyzing inhibition of growth and metastasis of tumor by ExoPep-KLA peptide in an A549 human lung cancer cell mouse tumor model.

FIG. 15 shows results of analyzing blood levels and liver and kidney function after treatment with ExoPep-KLA peptide in an A549 human lung cancer cell mouse tumor model.

FIG. 16 shows results of analyzing inhibition of growth and metastasis of tumor by co-administration of ExoPep-KLA peptide and doxorubicin in an A549 human lung cancer cell mouse tumor model.

FIG. 17 shows results of analyzing blood levels and liver and kidney function after co-administration of doxorubicin and ExoPep-KLA peptide in an A549 human lung cancer cell mouse tumor model.

FIG. 18 shows results of analyzing inhibition of growth and metastasis of tumor by administration of ExoPep-KLA peptide alone and co-administration of gemcitabine in a Panc-1 pancreatic cancer mouse model.

BEST MODE

An example embodiment of the present disclosure provides a peptide specifically binding to a cancer cell-derived exosome, having an amino acid sequence represented by SEQ ID NO: 1.

Preferably, the cancer cell may be a lung cancer cell, a breast cancer cell, or a pancreatic cancer cell, but is not limited thereto.

The peptide of an example embodiment of the present disclosure may be easily prepared by chemical synthesis known in the art (Creighton, Proteins; Structures and Molecular Principles, W. H. Freeman and Co., NY, 1983). Typical methods may include liquid or solid phase synthesis, fragment condensation, and F-MOC or T-BOC chemical methods (Chemical Approaches to the Synthesis of Peptides and Proteins, Williams et al., Eds., CRC Press, Boca Raton Florida, 1997; A Practical Approach, Athert on & Sheppard, Eds., IRL Press, Oxford, England, 1989), but are not limited thereto.

In addition, the peptide of an example embodiment of the present disclosure may be prepared by a genetic engineering method. First, a DNA sequence encoding the peptide is synthesized according to a conventional method. DNA sequences may be synthesized by PCR amplification using appropriate primers. Alternatively, the DNA sequence may be synthesized by standard methods known in the art, such as uses of an automatic DNA synthesizer (e.g., those sold by Biosearch or AppliedBiosystems). The constructed DNA sequence is inserted into a vector including one or more expression control sequences (e.g., promoter, enhancer, etc.) that are operatively linked to the DNA sequence to control the expression of the DNA sequence, so as to transform the host cell with the recombinant expression vector prepared therefrom. The prepared transformant is cultured under an appropriate medium and conditions to express the DNA sequence, thereby harvesting substantially pure peptides encoded by the DNA sequence from the culture. The harvesting may be performed by a method known in the art (e.g., chromatography). The term 'substantially pure peptide' as used herein may refer to a state that the peptide according to an example embodiment of the present disclosure does not substantially include any other proteins derived from the host.

In an example embodiment of the present disclosure, the peptide having the amino acid sequence represented by SEQ ID NO: 1 is a concept including a functional variant thereof. The term 'functional variant' as used herein refers to all similar sequences in which substitution of some amino acids takes place at a site of amino acids that do not affect the properties of the peptide of an example embodiment of the present disclosure specifically binding to a cancer cell-derived exosome.

In addition, an example embodiment of the present disclosure provides a polynucleotide encoding the peptide.

The term 'polynucleotide' as used herein refers to a polymer of deoxyribonucleotides or ribonucleotides that exist in single-stranded or double-stranded form. Including RNA genomic sequences, DNA (gDNA and cDNA), and RNA sequences transcribed therefrom, analogs of natural polynucleotides are included unless otherwise specified.

The polynucleotide includes not only the nucleotide sequence encoding the peptide, but also a sequence complementary to the sequence. The complementary sequence includes not only perfectly complementary sequences, but also substantially complementary sequences.

In addition, the polynucleotide may be modified. Such modifications include additions, deletions, or non-conservative substitutions or conservative substitutions of nucleotides. The polynucleotide encoding the amino acid sequence may be construed to include a nucleotide sequence showing substantial identity to the nucleotide sequence. The substantial identity may refer to a sequence showing at least 80% homology, at least 90% homology, or at least 95% homology derived by aligning the nucleotide sequence with any other sequences to the maximum correspondence and then analyzing the aligned sequence using an algorithm commonly applied in the art.

In addition, an example embodiment of the present disclosure provides a recombinant vector including the polynucleotide.

In addition, an example embodiment of the present disclosure provides a transformant (except for human) transformed with the recombinant vector.

In an example embodiment of the present disclosure, the term 'vector' as used herein refers to a self-replicating DNA molecule used to carry a clonal gene (or another piece of clonal DNA).

In an example embodiment of the present disclosure, the term 'recombinant vector' as used herein refers to a plasmid, viral vector, or other media known in the art that may express an inserted nucleic acid in a host cell, and may be one in which polynucleotides encoding the peptides of an example embodiment of the present disclosure may be operably linked to a conventional expression vector known in the art. The recombinant vector may include polynucleotides encoding the peptides of an example embodiment of the present disclosure operably linked to an origin of replication capable of generally proliferating in a host cell, one or more expression control sequences controlling expression (e.g., promoter, enhancer, etc.), a selective marker, and an expression control sequence. The transformant may be one transformed by the recombinant vector.

Preferably, the transformant may be obtained by introducing a recombinant vector including a polynucleotide encoding the peptide of an example embodiment of the present disclosure into a host cell by methods known in the art, for example, but not limited thereto, transient transfection, microinjection, transduction, cell fusion, calcium phosphate precipitation, liposome-mediated transfection, DEAE dextran-mediated transfection, polybrene-mediated transfection, electroporation, gene gun, and other known methods for introducing a nucleic acid into a cell (Wu et al., J. Bio. Chem., 267:963-967, 1992; Wu and Wu, J. Bio. Chem., 263:14621-14624, 1988).

In addition, an example embodiment of the present disclosure provides a composition for diagnosing cancer including the peptide as an active ingredient.

Preferably, the peptide may specifically bind to a cancer cell-derived exosome. More preferably, the cancer may include lung cancer, breast cancer, pancreatic cancer, brain tumor, liver cancer, skin cancer, esophageal cancer, testicular cancer, kidney cancer, colorectal cancer, rectal cancer, stomach cancer, bladder cancer, ovarian cancer, bile duct cancer, gallbladder cancer, uterine cancer, cervical cancer, prostate cancer, head and neck cancer, and squamous cell carcinoma, but is not limited thereto.

In an example embodiment of the present disclosure, the term 'diagnosis' as used herein refers to identifying the presence or characteristics of a pathological condition. For the purposes of an example embodiment of the present disclosure, diagnosis is to identify the presence or characteristics of cancer.

Diagnosis of cancer using the peptide of an example embodiment of the present disclosure may be conducted by reacting the peptide of an example embodiment of the present disclosure with tissues or cells directly obtained by blood, urine, or biopsy and detecting the binding thereof.

In addition, in order to easily identify, detect, and quantify whether the peptide of an example embodiment of the present disclosure binds to cancer tissues, the peptide of an example embodiment of the present disclosure may be provided in a labeled state. In other words, it may be provided by being linked (e.g., covalently bonded or cross-linked) to a detectable label. The detectable label may include a chromogenic enzyme (e.g., peroxidase, alkaline phosphatase), a radioactive isotope (e.g., $^{124}$I, $^{125}$I, $^{111}$In, $^{99}$mTc, $^{32}$P, $^{35}$S), a chromophore, a luminescent material, or a fluorescent material (e.g., FITC, RITC, rhodamine, cyanine, Texas Red, fluorescein, phycoerythrin, and quantum dots).

Similarly, the detectable label may be an antibody epitope, a substrate, a cofactor, an inhibitor, or an affinity ligand. Such labeling may be performed during the process of synthesizing the peptide of an example embodiment of the present disclosure or may be additionally performed on the already synthesized peptide. If a fluorescent substance is used as a detectable label, it is possible to diagnose cancer by fluorescence mediated tomography (FMT). For example, the peptide of an example embodiment of the present disclosure labeled with the fluorescent substance may be circulated in the blood to observe the fluorescence by the peptide via fluorescence tomography. If fluorescence is observed, cancer is diagnosed.

In addition, an example embodiment of the present disclosure provides a composition for drug delivery including the peptide as an active ingredient.

Preferably, the drug may be a peptide drug or an anticancer drug. More preferably, the peptide drug may be a cytotoxic peptide having activities of inducing apoptosis or necrosis, but is not limited thereto.

The peptide according to an example embodiment of the present disclosure may be used as an intelligent drug delivery that selectively delivers drugs to cancer cell-derived exosomes. Using the peptide of an example embodiment of the present disclosure for cancer treatment by linking the peptide with a conventionally known drug, it is possible to increase efficacy of the drug since the drug is selectively delivered to cancer cell-derived exosomes by the peptide of an example embodiment of the present disclosure, while the side effect of the drug on normal tissues may be significantly reduced.

The drug is an anticancer agent, and as an anticancer agent that may be linked to the peptide of an example embodiment of the present disclosure, it may be used without limitation as long as it is used for the conventional cancer treatment. Examples may include mertansine, doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, adriamycin, methotrexate, busulfan, chlorambucil, cyclophosphamide, melphalan, nitrogen mustard, and nitrosourea. The linkage between the anticancer agent and the peptide of an example embodiment of the present disclosure may be carried out by methods known in the art such as covalent bonding or crosslinkage. To this end, if necessary, the peptide of an example embodiment of the present disclosure may be chemically modified in a range that the activity thereof is not degraded.

In addition, an example embodiment of the present disclosure provides a fusion peptide in which an apoptosis-inducing peptide having an amino acid sequence represented by SEQ ID NO: 2 is bound to the peptide.

The apoptosis-inducing peptide having the amino acid sequence represented by SEQ ID NO: 2 is 'KLAKLAKKLAKLAK', which is abbreviated as 'KLA' in the present specification.

In addition, an example embodiment of the present disclosure provides a pharmaceutical composition for preventing or treating cancer including the fusion peptide as an active ingredient.

In addition, an example embodiment of the present disclosure provides a pharmaceutical composition for preventing or treating cancer including the fusion peptide and an anticancer agent as active ingredients.

Preferably, the anticancer agent may include mertansine, doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, adriamycin, methotrexate, busulfan, chlorambucil, cyclophosphamide, melphalan, nitrogen mustard, or nitrosourea, but is not limited thereto.

Preferably, the cancer may include lung cancer, breast cancer, pancreatic cancer, brain tumor, liver cancer, skin cancer, esophageal cancer, testicular cancer, kidney cancer, colorectal cancer, rectal cancer, stomach cancer, bladder cancer, ovarian cancer, bile duct cancer, gallbladder cancer, uterine cancer, cervical cancer, prostate cancer, head and neck cancer, and squamous cell carcinoma, but is not limited thereto.

The pharmaceutical composition of an example embodiment of the present disclosure may be prepared by using a pharmaceutically suitable and physiologically acceptable adjuvant in addition to the active ingredient, and a solubilizing agent such as an excipient, a disintegrant, a sweetener, a binder, a coating agent, an expanding agent, a polisher, a lubricant, or a flavoring agent may be used as the adjuvant. The pharmaceutical composition of an example embodiment of the present disclosure may be preferably formulated into a pharmaceutical composition by including one or more pharmaceutically acceptable carriers in addition to the active ingredient for administration. As an acceptable pharmaceutical carrier for a composition formulated as a liquid solution, components should be sterilized and biocompatible and may be used by mixing saline, sterile water, Ringer's solution, buffered saline, albumin injection, dextrose solution, maltodextrin solution, glycerol, ethanol and one or more of these components. Other conventional additives such as antioxidants, buffers, and bacteriostats may be added if needed. In addition, diluents, dispersants, surfactants, binders, and lubricants may be additionally added to be formulated into an injectable formulation such as an aqueous solution, suspension, and emulsion, pills, capsules, granules, or tablets.

The pharmaceutical formulation type of the pharmaceutical composition of an example embodiment of the present disclosure may include granules, powder, coated tablets, tablets, capsules, suppositories, syrups, juices, suspensions, emulsions, drops or injectable solutions, and sustained-release formulations of an active compound. The pharmaceutical composition of an example embodiment of the present disclosure may be administered in a conventional manner via intravenous, intraarterial, intraperitoneal, intramuscular, intraarterial, intraperitoneal, intrasternal, transdermal, intranasal, inhalational, topical, rectal, oral, intraocular, or intradermal routes. The effective dose of the active ingredient of the pharmaceutical composition of an example embodiment of the present disclosure refers to an amount required for preventing or treating a disease. Therefore, the dose may be adjusted by the type of disease, the severity of the disease, the type and content of the active ingredient and other ingredients included in the composition, the type of formulation, age, weight, general health condition, sex and diet of the patient, administration time, administration route, secretion rate of the composition, treatment period, and various factors including drugs that are used in combination with. Although not limited thereto, in the case of an adult when administered once to several times a day, the dose of the composition of an example embodiment of the present disclosure may be, for example, 0.1 ng/kg-10 g/kg in the case of a compound.

In addition, an example embodiment of the present disclosure provides a composition for imaging cancer cells including the peptide as an active ingredient.

Preferably, the peptide may be labeled with a chromogenic enzyme, a radioisotope, a chromophore, a luminescent material, a fluorescer, a magnetic resonance imaging material, superparamagnetic particles, or ultrasuper paramagnetic particles, but is not limited thereto.

Cancer cell imaging and cancer diagnosis may be used, but are not limited thereto, not only for the purpose of initial diagnosis of a cancer disease, but also for monitoring progression, treatment, and response to a therapeutic agent. The peptide may be provided in a labeled state in order to facilitate identification, detection, and quantification of binding, which is the same as described above.

In addition, an example embodiment of the present disclosure provides a composition for detecting a cancer cell-derived exosome, including the peptide as an active ingredient.

MODES FOR CARRYING OUT INVENTION

Hereinafter, example embodiments will be described in detail to help the understanding of the present disclosure. However, the following example embodiments are merely illustrative of the content of the present disclosure, and the scope of the present disclosure is not limited to the following examples. The example embodiments of the present disclosure are provided to more completely explain the present disclosure to those of ordinary skill in the art.

<Example 1> Analysis of characteristics of exosomes isolated from A549 tumor cells

Exosomes were isolated from the A549 lung cancer cell medium using an ultracentrifuge or Exoquick, a commercial reagent. First, in order to analyze the characteristics of the isolated exosomes by western blotting, an exosome extract was loaded on SDS polyacrylamide gel. The gel was electrophoresed and transferred to an NC membrane, and a reaction was carried out for 1 hour using a blocking solution composed of Tris buffer (TBST) components containing 5% skim milk and Tween-20 so as to block non-specific reactions. Thereafter, the membrane was washed with TBST 3 times for 10 minutes and reacted with antibody (Abcam, USA) against CD63 and Alix overnight at 4°C. The next day, the membrane was washed several times with TBST at room temperature, and a reaction was carried out with horse radish peroxidase-conjugated secondary antibody for 1 hour at room temperature. Thereafter, analysis was conducted using West Femto Maximum Sensitivity Substrate (ThermoFisher, USA) reagent and LAS.

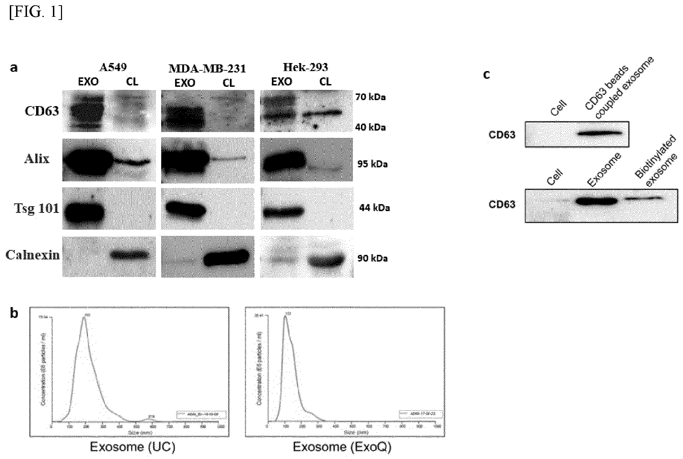

As a result, exosomes derived from A549, MDA-MB231, and HEK293 cell lines showed higher quantitative levels of exosome markers such as CD63, Alix, and Tsg 101, compared to cell extracts, and calnexin was expressed only in the cell extract (FIG. 1A). On the other hand, there was no significant difference in the level of exosome markers according to cell lines.

In addition, in order to measure the size of the isolated exosomes, the exosomes were analyzed using a NanoSight instrument. As a result, the sizes of the exosomes isolated via ultracentrifugation and Exoquick were 193 nm and 103 nm, respectively, which were in the normal size range of exosomes (FIG. 1B).

In addition, exosomes captured by CD63 antibody-labeled beads (Invitrogen, USA) and exosomes captured by avidin magnetic beads after labeled with biotin were subjected to gel electrophoresis and Western blotting using CD63 antibody in the same manner as described above. As a result, the exosome marker CD63 was well observed in both cases (FIG. 1C).

<Example 2> Excavation of peptides specifically binding to A549 tumor cell-derived exosomes through phage screening

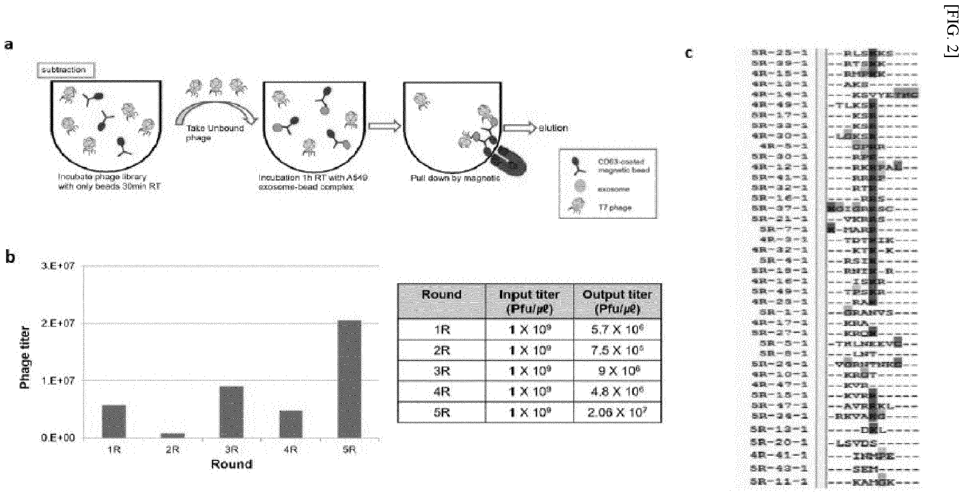

Phage screening was performed using a T7 phage hydrophobic amino acid library ($1.3 \times 10^{10}$ pfu). The library has a CXXXXXXXC (X = random sequences) amino acid sequence having cysteines at both ends and 7 random amino acids in the middle while at least one of the 7 random amino acids has hydrophobic amino acid. A549 was used as a lung cancer cell line. Exosomes were isolated from the tumor cell medium using an ultracentrifuge. In order to select T7 phages selectively binding only to A549-derived exosomes, a process of removing phages that non-specifically bind to the beads was performed first, by reacting the phages with CD63 antibody-labeled beads, and then collecting the phages in the supernatant that were not bound to the beads. Then, the phage in the supernatant was reacted with the exosomes captured on the CD63 antibody-labeled beads (FIG. 2A).

Phages bound to the exosomes were collected using E. coli as a host, and the titer of phages collected in each round was calculated via a plaque assay. Remaining phages unsubjected to the plaque assay were amplified using E. coli to secure phages to be used in the next round. Same processes were repeated up to 5 rounds.

As a result, the titer of phage collected at each round was calculated. When first round was performed, the titer of collected phages was about $5.7 \times 10^6$, and it was found that $2.06 \times 10^7$ phages were finally collected after 5 rounds. In other words, as a result of proceeding from round 1 to round 5, the titer of phage was increased by approximately 4 times (FIG. 2B).

For amino acid sequencing of peptides displayed on the selected phage, 50 clones (total of 100) were collected from the product of the plaque assay used for titer measurements in rounds 4 and 5 and stored in 10 ul of Tris buffer. The gene encoding the peptide inserted into these phages was amplified by PCR, the nucleotide sequence was analyzed, and the amino acid sequencing thereby was performed. The peptide sequence was aligned and analyzed using Clustal X program (FIG. 2C). Thereamong, 10 phage clones representing peptides composed of 7 to 9 amino acids were selected for further study.

<Example 3> Evaluation of selective binding ability of phage clones to A549 tumor cell-derived exosomes and exosome-producing cells

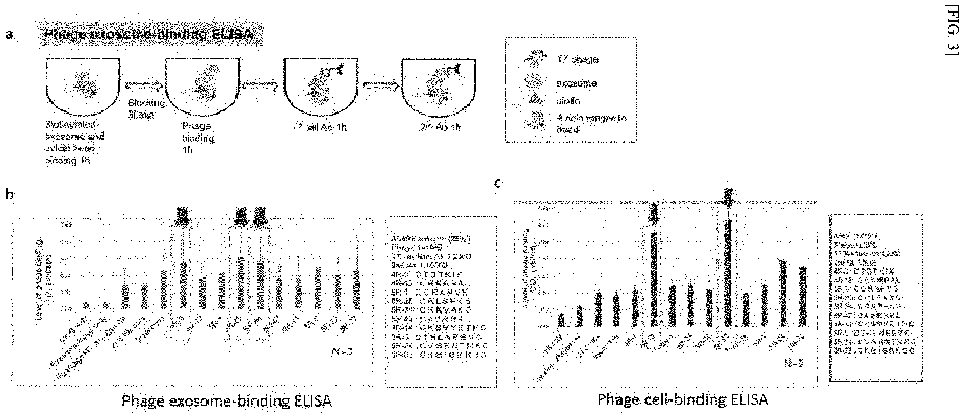

To investigate the selective binding of phage clones to A549 cell-derived exosomes and exosome-producing cells, phage exosome binding ELISA and phage cell binding ELISA were performed. For phage exosome binding ELISA, exosomes were first biotinylated and immobilized by monomeric avidin-labeled magnetic beads (FIG. 3A). Phage bound to the exosome was detected with an antibody against horseradish peroxidase-conjugated T7 phage, and the amount was quantified according to the degree of response and color development using a substrate for the enzyme.

As a result, it was found that three phage clones each displaying peptides having the sequences of CTDTKIK (4R-3), CRLSKKS (5R-25), and CRKVAKG (5R-34) bound more to A549-derived exosomes than other clones (FIG. 3B).

In addition, in order to measure the binding of phage to the cells producing the exosomes through phage cell binding ELISA, the cells were cultured on a plate, and the phages bound to the cells were quantified using antibody and substrate as described above. As a result, it was found that two clones each displaying peptides having the sequences of CRKRPAL (4R-12) and CAVRRKL (5R-47) bound more to exosome-producing A549 cells than other clones (FIG. 3C).

<Example 4> Analysis of binding of ExoPep peptide to A549 tumor cell-derived exosomes and internalization thereof

5 types of peptides (CTDTKIK, CRLSKKS, CRKVAKG, CRKRPAL, CAVRRKL) were synthesized based on the result of ELISA shown in FIG. 3. In order to investigate the binding of candidate peptides by FACS method, a peptide in which fluorescein isothiocyanate (FITC), a fluorescent substance, is conjugated to the carboxy terminus was synthesized by requesting Peptron Co. (Daegeon, Korea). Each peptide was synthesized by standard Fmoc method and purified by mass spectrometry. In addition, FITC-labeled NSSSVDK peptide was used as a control.

In order to observe the peptide binding to the exosomes, biotin and exosomes were first combined in the same manner as described in FIG. 3, and then the exosomes were immobilized by binding with avidin beads. The exosome-bead complex was blocked for non-specific binding through treatment of 1% bovine serum albumin (BSA) dissolved in phosphate buffer for 30 minutes at room temperature. After washing, the FITC-labeled peptide was bound to the exosome-bead complex at 4°C for 30 minutes. After the reaction, the binding of the peptide to the exosomes was analyzed using a flow cytometer.

On the other hand, in order to observe the peptide binding to the exosome-producing cells, $1 \times 10^6$ cells were first prepared. Each cell medium was treated with 1% BSA at 37°C for 30 minutes. Thereafter, the FITC-labeled peptide was bound at 4°C for 1 hour. After the reaction, the binding of the peptide to the cells was analyzed using a flow cytometer.

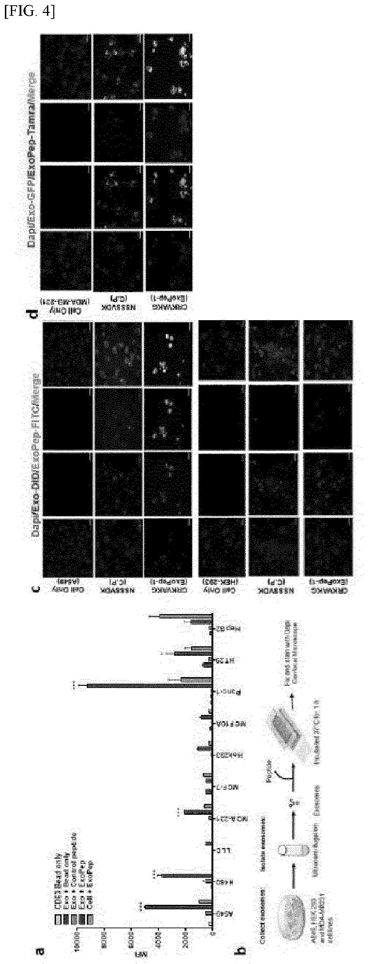

As a result, as shown in FIG. 4A, the peptide (sequence: CRKVAKG, name: ExoPep; SEQ ID NO: 1) of an example embodiment of the present disclosure showed higher binding to exosomes derived from A549 lung cancer cells, H460 lung cancer cells, MDA-MB231 breast cancer cells, and Panc-1 pancreatic cancer cells, compared to each exosome-producing cell. On the other hand, in exosomes derived from normal cells including HEK293 and MCF10A cells and other types of tumor cells including LLC mouse lung cancer cells, HT-29 colorectal cancer cells, and HepG2 liver cancer cells showed very low binding, or no higher binding was observed as compared to each exosome-producing cells. On the other hand, other types of peptides were excluded from the candidate group due to relatively high binding to normal cells.

FIG. 4B shows a schematic diagram of the experiment for the binding and internalization of exosomes and ExoPep peptides. Media of A549, HEK293, and MDA-MB231 cells were collected, exosomes were isolated through ultracentrifugation, and exosomes were labeled with DiD fluorescence reagent (red), followed by a reaction with ExoPep peptide (FITC label, green). After the reaction, the cells were immobilized, and the nuclei were stained with DAPI (blue) to analyze with confocal microscopy. More specifically, the isolated exosomes and DiD (1:200 dilution) were reacted on a stirrer at 37°C for 30 minutes. After adding Exoquick reagent, condition was maintained at 4°C for 30 minutes, and then DiD-labeled exosomes were precipitated by centrifugation at 13000 rpm for 3 minutes. Thereafter, a mixture of 10 uM FITC-conjugated peptide and DiD-labeled exosomes was reacted with A549 cells at 37°C for 1 hour. After washing three times, immobilization was performed using 2% paraformaldehyde (PFA). After washing again, cells were treated with 4'-6-diamidino-2-phenylindole (DAPI) for nucleus staining. Finally, after mounting, observation was followed using a confocal microscope (Zeiss, Oberkochen, Germany).

As a result, the ExoPep peptide was observed along with the exosomes in A549 cells, but the control peptide (c.p) was not observed (FIG. 4C). This suggests that the control peptide did not bind to the A549 cell-derived exosome, but the ExoPep peptide was bound and effectively internalized into the cell. On the other hand, the exosomes derived from normal HEK293 cells were treated in the same manner as described above and observed under a confocal microscope (Zeiss, Oberkochen, Germany). As a result, the internalization of exosomes into HEK293 cells occurred effectively, while the control peptide or the ExoPep peptide was not observed in the cells (FIG. 4C). This suggests that ExoPep peptide did not bind to exosomes of normal cells.

In addition, an experiment was performed on exosomes derived from MDA-MB231 cells which were prepared to secrete exosomes showing green fluorescence by expressing CD63-GFP. GFP exosomes (green) and 10 uM TAMARA-conjugated peptide (red) were reacted with MDA-MB231 cells at 37°C for 1 hour. Thereafter, after performing immobilization, DAPI nucleus staining, and mounting as described above, observation was followed using a confocal microscope (Zeiss, Oberkochen, Germany). As a result, the ExoPep peptides bound to the MDA-MB231 cell-derived exosome and then were internalized into the cell along with the exosome, more of which were observed in the cells than the control peptide (FIG. 4D).

<Example 5> Inhibition of internalization of exosomes into recipient cells using the binding of ExoPep peptides to A549 tumor cell-derived exosomes

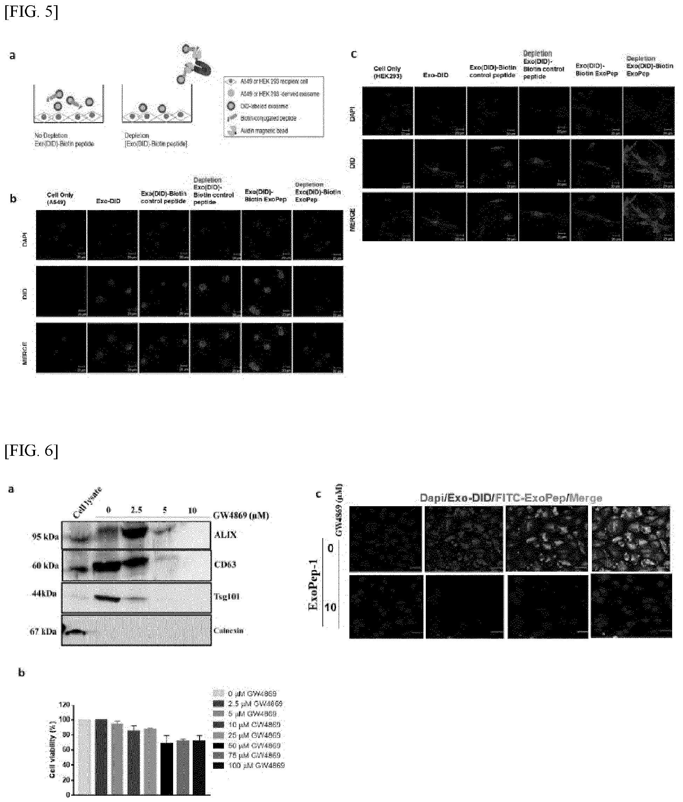

In order to further identify the binding specificity of the ExoPep peptide to the A549 cell-derived exosome, the exosomes were labeled with a DID fluorescent dye, reacted with the biotin-labeled ExoPep peptide, and then conjugated with biotin by reacting the labeled avidin with magnetic particles. The complex of exosomes/biotin-peptide/avidin-magnetic particles prepared by the reaction was captured and removed using a magnet, and the remaining exosomes were treated to recipient cells (FIG. 5A).

As a result, compared to the case that the cell-derived exosomes were directly treated to the recipient cells or the exosomes and the ExoPep peptide were reacted without undergoing a removal process, internalization of the exosomes into the recipient cells was significantly inhibited by pretreating the A549 cell-derived exosomes with the ExoPep peptide and then removing the same (FIG. 5B). On the other hand, internalization of exosomes into recipient cells was not reduced by pretreatment with the control peptide (sequence: NSSSVDK) (FIG. 5B). In addition, when a similar experiment was conducted on exosomes derived from normal HEK293 cells, the internalization of exosomes into recipient cells was not inhibited by the ExoPep peptide of an example embodiment of the present disclosure as well as the control peptide (FIG. 5C).

<Example 6> Reduction in exosome binding and internalization of ExoPep peptide due to inhibition of release of A549 tumor cell-derived exosomes

In order to further identify the binding specificity of ExoPep to exosomes, GW4869 (sphingomyelin inhibitor, Sigma-Aldrich), a drug that inhibits the release of exosome out of cells, was treated to cells at various concentrations (2.5, 5, and 10 $\mu$M). Thereafter, exosomes were isolated, and electrophoresis and western blotting with antibodies against CD63, Alix, Tsg101, and calnexin (Abcam) were performed in the same manner as described in FIG. 1.

As a result, it was found that exosome markers such as CD63, Alix, and Tsg101 were greatly reduced at a concentration of 10 $\mu$M GW4869 inhibitor compared to concentrations of 2.5 and 5 $\mu$M (FIG. 6A).

On the other hand, in order to identify the effect of the GW4869 inhibitor on the cell viability, A549 cells ($5 \times 10^3$ cells per well in a 96-well cell culture vessel) were cultured in serum-free medium with various concentrations of GW4869 at 37°C for 3 hours. Thereafter, the medium was replaced with a culture medium containing 10% bovine serum (FBS), cells were cultured for 24 hours, and then cytotoxicity was measured using CCK-8 assay (Dojindo, Japan). As a result, GW4869 did not show toxicity even at high concentration (FIG. 6B). Based on the above results, using 10 μM GW4869 inhibitor, the binding of the ExoPep peptide to the exosomes and internalization thereof were analyzed. As a result of immunofluorescence staining, when 10 μM GW4869 was treated, internalization of exosomes and peptides bound therewith was significantly reduced due to decreased release of exosomes (FIG. 6C).

<Example 7> Analysis of binding and internalization of ExoPep peptide to exosomes derived from A549 tumor mouse blood and normal mouse blood

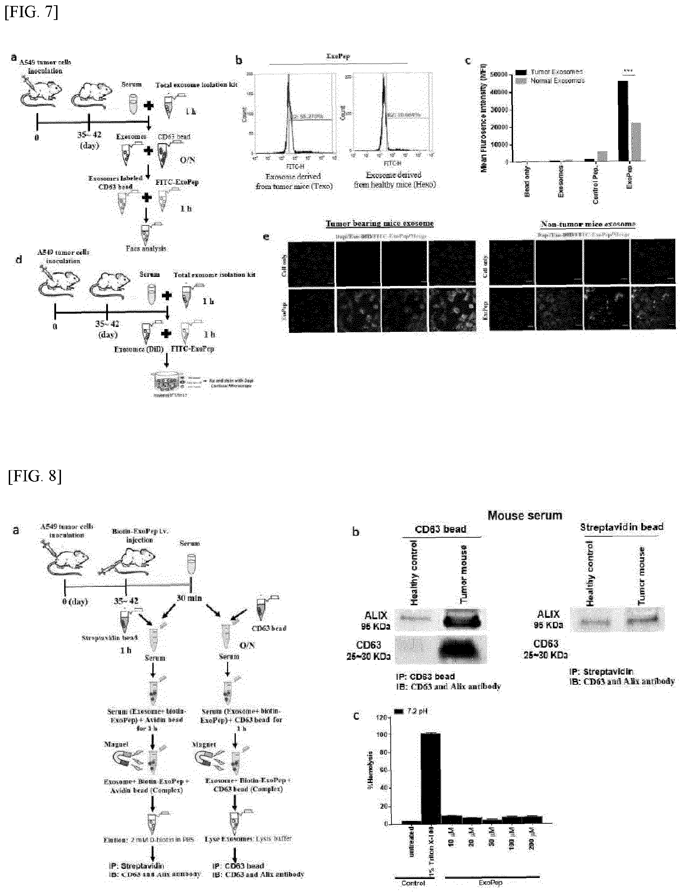

A549 cells were injected into BALB/c nude mice, blood was collected after the tumor grew, and exosomes were isolated using an exosome isolation kit. To measure the binding of the peptide to the exosomes by flow cytometry, the exosomes were labeled with CD63 antibody beads. As a control group, exosomes isolated from the blood of normal mice were used. The exosome-bead complex labeled with CD63 beads was blocked by a reaction with 1% BSA/PBS at room temperature for 30 minutes to reduce non-specific binding. After washing, the FITC-labeled ExoPep peptides were bound to the exosome-bead complex at 4°C for 30 minutes. After washing again with PBS, the binding of the peptide to the exosome-bead complex was analyzed using a flow cytometer (ThermoFisher Scientific, USA) (FIG. 7A).

As a result, it was found that the ExoPep peptide bound better to the A549 tumor mouse blood-derived exosome than the normal mouse blood-derived exosome (FIGS. 7B AND 7C).

In order to observe internalization of peptides into cells, exosomes were labeled with DiD, reacted with FITC-labeled peptides, and treated to A549 cells. Cells were immobilized with 4% paraformaldehyde for 5 minutes and stained with DAPI, followed by observation under a microscope.

As a result, it was found that the ExoPep peptide of an example embodiment of the present disclosure bound to the tumor mouse blood-derived exosome and were internalized more into the recipient cell A549 than the normal mouse blood-derived exosome. In addition, the fluorescence of the peptide (green) and tumor-derived exosomes (red) was observed together in the cells (FIG. 7E).

<Example 8> Analysis of binding of ExoPep peptide to exosomes circulating in blood in A549 tumor mice

In order to check the binding of peptides with exosomes circulating in mouse blood, biotin-labeled peptides were injected intravenously into A549 tumor mice and healthy mice and circulated. Then, serum was collected, and reactions were carried out with CD63 antibody beads and streptavidin beads respectively 30 minutes after the collection. Serum treated with CD63 antibody beads was reacted overnight, and the beads were precipitated using a magnet the next day. Exosomes bound to beads were lysed using RIPA lysate, and then Western blot analysis was performed using CD63 and ALIX antibodies. Serum treated with streptavidin beads was reacted for 1 hour, and then precipitated using a magnet. Exosomes bound to the beads were eluted using an elution buffer (2 mM D-biotin in PBS), and then Western blot analysis was performed using CD63 and ALIX antibodies (FIG. 8A).

As a result, compared to healthy mice, exosome markers were detected much higher when isolated with CD63 antibody beads in the blood of tumor mice (FIG. 8B, left). Even when isolated with the streptavidin beads, exosome markers were detected higher in the blood of tumor mice, but the amount of exosome markers was relatively lower than when isolated with the CD63 beads (FIG. 8B, right). This suggests that the amount of isolated exosomes was relatively low since the CD63 antibody bound to all exosomes in the blood while the biotin-ExoPep peptide selectively bound to the tumor-derived exosomes. The above results shows that the peptide of an example embodiment of the present disclosure bound to exosomes in circulating blood, and in particular, selectively bound to tumor-derived exosomes.

Next, an in vitro hemolysis assay was performed. In vitro hemolysis assay, as an indicator of hemolysis of red blood cells after exposure to a drug or an agent, was conducted by measuring the hemoglobin release in plasma. This is an accurate, sensitive method to predict the hemolytic activity of a drug. Fresh blood was centrifuged at 500×g for 10 minutes, and the pellet of red blood cells was washed 3 times and re-suspended in 10 mM PBS in pH 7.4. Equal volumes of red blood cells were reacted with various concentrations of ExoPep peptide by stirring at 37°C for 1 hour. The samples were then centrifuged at 4°C at 500×g for 10 minutes. RBC lysis was measured by analyzing absorbance at OD 540 nm depending on the concentration of different peptides. Considering that a 1% Triton X 100 sample used as a control shows 100% hemolysis, the percent hemolysis was measured. The hemolytic activity of the peptide was calculated in a percentage using the following equation.

$$\text{Equation: } H = 100 \times (O_P - O_B)/(O_T - O_B)$$

In the equation above, $O_P$, $O_B$, and $O_T$ represent optical density of the peptide solution at an indicated concentration, optical density of a buffer, and optical density of Triton X 100, respectively.

As a result, only low levels of hemolytic activity were shown at various concentrations of peptides up to a final concentration of 200 μM (FIG. 8C).

<Example 9> In vivo distribution of A549 tumor mouse blood-derived exosomes and A549 cell-derived exosomes in A549 tumor mice depending on the binding of ExoPep peptide

DiD-labeled A549 tumor mouse blood-derived exosomes and A549 cell-derived exosomes were reacted with ExoPep peptide, and image monitoring was performed for in vivo distribution. Tumor xenograft mice were prepared by subcutaneously implanting A549 cell suspension ($5 \times 10^6$ cells) along with PBS into the right flank of 5-week-old female BALB/c nude mice. When the tumor size reached a volume of approximately 100-200 mm$^3$, the mice were anesthetized. A549 tumor mice was intravenously administered with DiD-labeled A549 tumor mouse blood-derived exosomes (mEXO) and a product obtained by reacting the exosomes (mEXO) with peptides (mEXO+ExoPep, n = 3) as well as DiD-labeled A549 cell-derived exosomes (cEXO) and a product obtained by reacting the exosomes (cEXO) with peptides (cEXO+ExoPep, n = 3). The inventors of the present disclosure used FITC-labeled control peptide and ExoPep (n = 3) as controls. In vivo fluorescence images were analyzed using an IVIS imaging system (Caliper Sciences, Massachusetts, USA) at various time points (2, 4, 8, and 24 hours, respectively) after administration of DiD-labeled exosomes. In vitro fluorescence images of excised tumors and organs were collected 24 hours after administration. All major organs (liver, kidney, spleen, heart, and lung) having tumor tissues were separated, washed with PBS, and subjected to in vitro fluorescence analysis (n = 3).

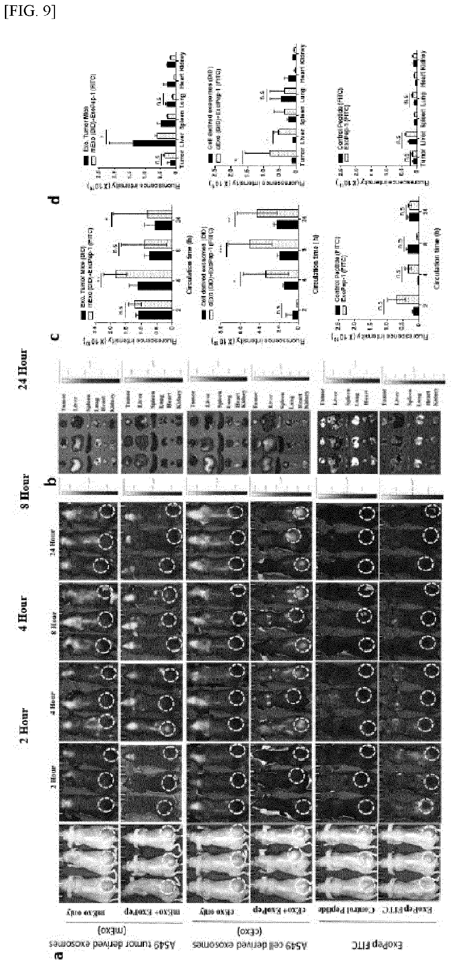

As a result, compared to mEXO and cEXO, it was observed that the combination of mEXO+ExoPep and cEXO+ExoPep was accumulated more in the tumor tissues 4 hours after injection (FIG. 9A). Statistical significance was also derived in the result of analyzing the fluorescence intensity of a region of interest (ROI) in the entire body, specifically, a tumor site (FIG. 9C). 24 hours after injection, organs were removed and fluorescence was photographed (FIG. 9B). As a result of measuring the intensity, there was no difference in the accumulation of mEXO+ExoPep in the tumor tissue compared to mEXO, but less accumulation was observed in the liver (FIG. 9D). On the other hand, compared to cEXO, it was shown that cEXO+ExoPep was accumulated more in the tumor tissues and liver (FIG. 9D). The FITC-labeled control and the ExoPep peptide itself showed low signals (FIGS. 9A-D).

<Example 10> Cytotoxic IC50 analysis of ExoPep-KLA peptide composed of ExoPep peptide and apoptosis-inducing peptide

Using the fact that ExoPep peptide is well internalized into the cell after binding with exosomes, a peptide (named as ExoPep-KLA) was prepared, fused with a peptide (KLA) that induces apoptosis by causing damage to the mitochondrial membrane in the cell. To identify the cytotoxicity of ExoPep-KLA in A549, MDA-MB231, HEK293, Panc-1, HT29, and HepG2 cells ($5 \times 10^3$ cells per well in a 96-well cell culture vessel), the cells were cultured in serum-free medium with various concentrations of ExoPep-KLA at 37°C for 3 hours. Thereafter, the medium was replaced with a medium containing 10% FBS, the cells were cultured for 24 hours, and then cytotoxicity was measured using CCK-8 assay (Dojindo). Various concentrations of ExoPep-KLA were reacted with each exosome (5 μg) isolated from A549, MDA-MB231, Panc-1, HT29, HepG2, and HEK293 cells, and then treated to the cells. Used as a control was ExoPep-KLA alone or a product obtained by treating cells with ExoPep-KLA first and then with exosomes.

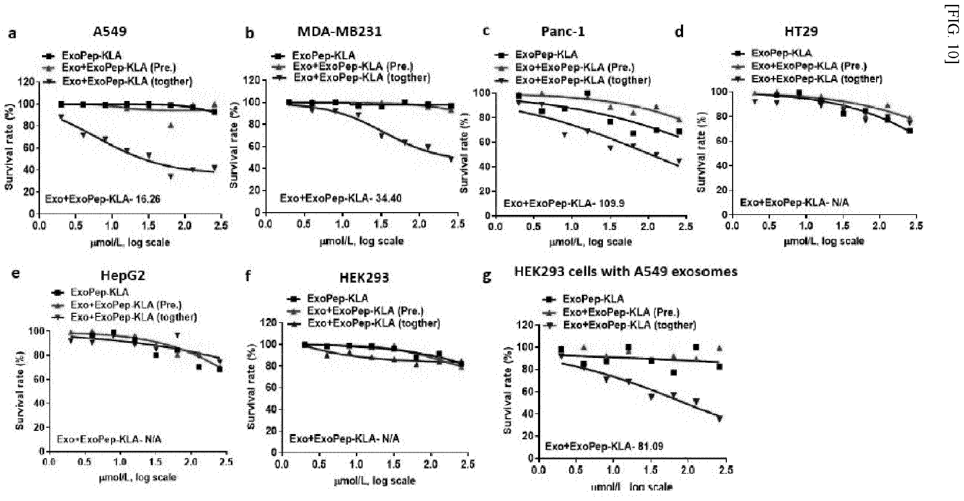

As a result, more effective cytotoxicity (i.e., lower IC50 value) was shown in the group treated with the peptide and the exosomes, compared to the group treated with the peptide alone and the group treated with the exosome after the peptide treatment. In particular, ExoPep-KLA peptide barely showed cytotoxicity in normal HEK293 cells, HT-29 colorectal cancer cells, and HepG2 liver cancer cells, but showed higher cytotoxicity specifically in A549 lung cancer cells, MDA-MB231 breast cancer cells, and Panc-1 pancreatic cancer cells (FIGS. 10A-F). On the other hand, after reacting exosomes of normal HEK293 cells with ExoPep-KLA, cytotoxicity was barely observed when treated to HEK293 cells. On the other hand, after reacting the A549 cell exosome with ExoPep-KLA and treating the same to HEK293 cells, cytotoxicity to HEK293 cells was derived when the exosome and ExoPep-KLA peptides were treated together, compared to the group treated with peptide alone and the group treated with the exosome after the peptide treatment (FIG. 10G). This indicates the binding specificity of the ExoPep-KLA peptide to exosomes derived from tumor cells.

<Example 11> Apoptosis-inducing effect of ExoPep-KLA

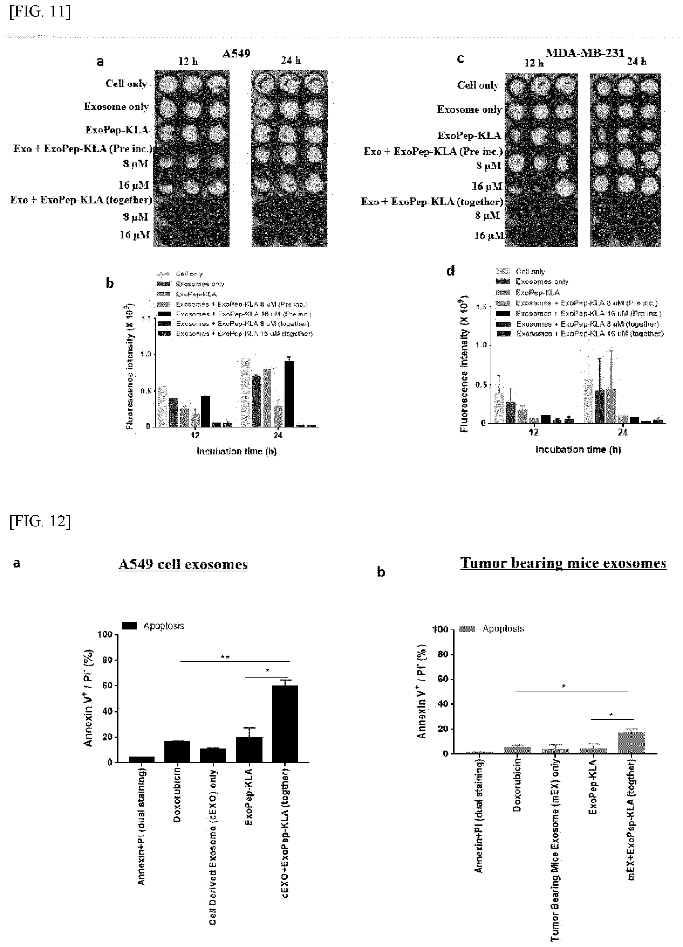

To investigate the induction of apoptosis by ExoPep-KLA, phosphorescent A549-luc and MDA-MB231-luc cells were inoculated at a density of $1 \times 10^4$ cells/well on a black 96-well ELISA plate. After reacting cells with exoPep-KLA along with exosomes (5 μg) of each cell for 24 hours, 3 μl (3 mg/mL) of D-luciferin was added to each well, and IVIS imaging system (PerkinElmer, Waltham, MA, USA) was used to measure phosphorescence intensity (efflux activity). As a result, compared to the group treated with ExoPep-KLA peptide alone and the group treated with exosomes after peptide treatment, the apoptosis effect was increased and the intensity of luc signal was decreased as the treatment concentration and reaction time increased by ExoPep-KLA reacted with exosomes (FIG. 11).

<Example 12> Apoptosis effect of ExoPep-KLA, subjected to reactions with A549 cell-derived exosomes and A549 tumor mouse blood-derived exosomes, on A549 cells

In order to identify the apoptosis-inducing effect of ExoPep-KLA depending on the binding with A549 cell-derived exosomes, the inventors of the present disclosure treated A549 cells with 5 μg of ExoPep-KLA pre-reacted with cell-derived exosomes or unreacted ExoPep-KLA. Staining was performed with Annexin V-647, and cells were analyzed using a flow cytometer (ThermoFisher Scientific, USA) to quantify apoptotic cells (Annexin+/PI-). ExoPep-KLA group (cEXO+ExoPep-KLA) pre-reacted with exosomes showed a higher proportion of apoptotic cells, 62.9% and 20.3%, respectively, compared to the group treated with ExoPep-KLA alone (p < 0.05, FIG. 12A). The results showed that ExoPep-KLA bound to exosomes induced apoptosis more effectively than ExoPep-KLA alone.

In addition, in order to measure the apoptosis-inducing effect of ExoPep-KLA depending on the binding with the mouse blood-derived exosomes subcutaneously implanted with A549 tumor, the inventors of the present disclosure treated A549 cells with mouse blood-derived exosomes (5 μg) implanted with A549 tumor with or without undergoing a pre-reaction with ExoPep-KLA. Staining was performed with Annexin V-647, and the cells were analyzed by flow cytometry (ThermoFisher Scientific, USA) to quantify apoptotic cells (Annexin+/PI-). The ExoPep-KLA group (mEXO+ExoPep-KLA) pre-reacted with the exosomes showed a higher apoptotic cell ratio, 19.1% and 6.1%, respectively, than the group treated with ExoPep-KLA alone, but the ratio itself was not high (p < 0.05, FIG. 12B). The above results shows that the greater number of ExoPep-KLA bound to tumor cell-derived exosomes than to mouse blood-derived exosomes implanted with tumors, and apoptosis was induced higher than in A549 cells.

<Example 13> Analysis of the stability of ExoPep-KLA in serum

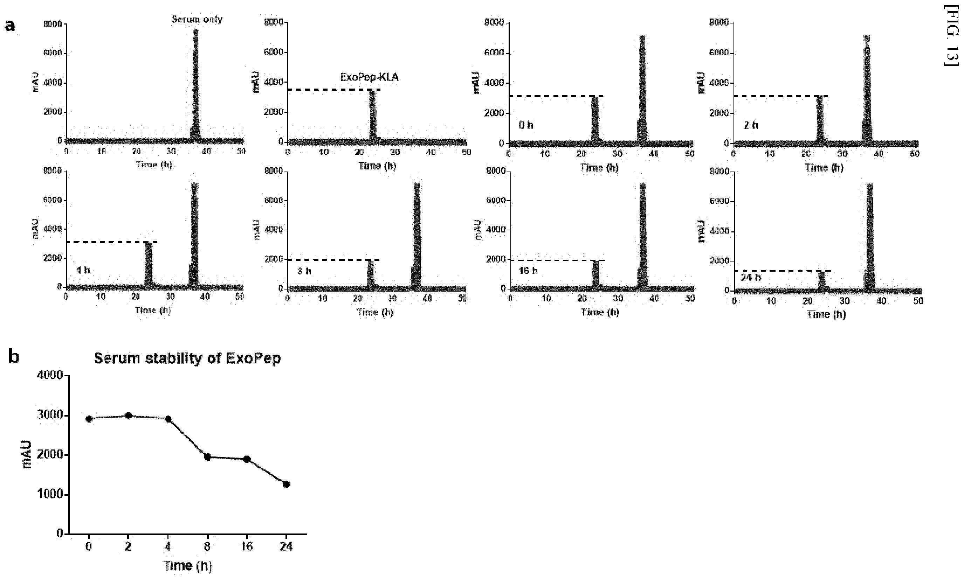

The inventors of the present disclosure tried to identify the stability of the ExoPep-KLA peptide in mouse serum. After the ExoPep-KLA peptides were reacted with mouse serum for 24 hours, the amount of peptides remaining in the serum was analyzed. The peptide peak was isolated from the non-specific peak of serum, and the residual amount of peptide in the serum was calculated through the peak area. ExoPep-KLA peptides were barely degraded until 4 hours and partially degraded at 8 hours, wherein a half-life was about 24 hours (FIGS. 13A AND 13B). As a result of mass spectrometry analysis for each peptide peak, it was found that the peak was the ExoPep-KLA peptide. The results showed that the peptide was relatively stable in serum.

<Example 14> Inhibition of growth and metastasis of tumor by ExoPep-KLA peptide in an A549 tumor mouse model

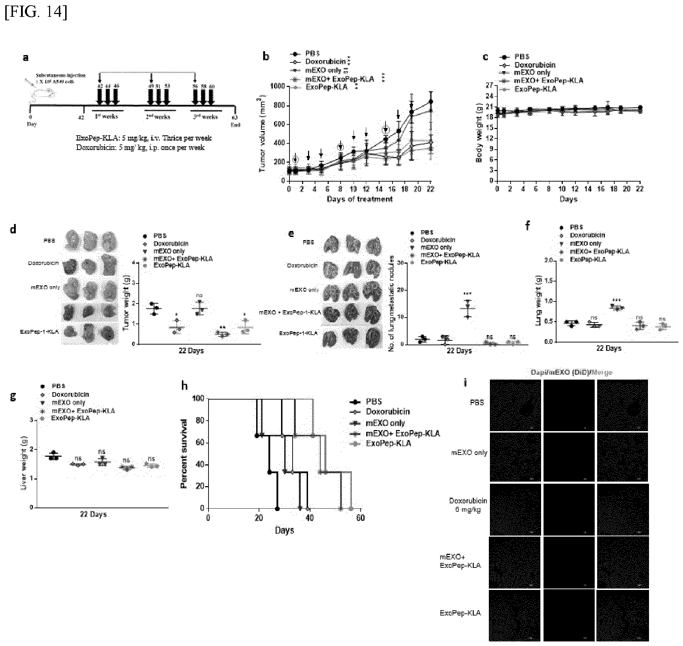

To test anticancer effect using ExoPep-KLA in the A549 tumor nude mouse model, ExoPep-KLA peptides were pre-reacted with exosomes either alone or in vitro and then systemically administered intravenously for 3 weeks at the time points indicated in the protocol, and doxorubicin was administered once a week for 3 weeks (FIG. 14A).

As a result, when administered with phosphate buffer and tumor mouse blood-derived exosome (mEXO only), tumor growth was not inhibited. However, tumor growth was significantly inhibited in the group administered with doxorubicin, ExoPep-KLA, and mEXO+ExoPep-KLA prepared by pre-reacting exosomes (mEXO) and ExoPep-KLA. In particular, even when ExoPep-KLA was injected alone, tumor growth was inhibited at a similar level compared to when exosomes were injected after a pre-reaction in vitro. This suggests that the ExoPep-KLA peptides bound to the tumor-derived exosome (mEXO) circulating in the blood and effectively migrated to the tumor tissues. The body weight of the mice was not changed during the treatment period even with continuous administration of the treatment group (FIG. 14C). On the other hand, the weight of tumor was reduced when doxorubicin, ExoPep-KLA, and mEXO+ExoPep-KLA were treated (FIG. 14D).

When A549 lung cancer cells were xenografted under the skin of nude mice, the number of tumor nodules due to lung metastasis was low (FIG. 14E). In addition, in the mEXO+ExoPep-KLA group and the group administered with ExoPep-KLA alone, the number of tumor nodules was slightly reduced compared to phosphate buffer and doxorubicin, but there was no significant difference (FIG. 14E). In addition, there was no significant difference in the weight of lung from other groups (FIG. 14F). However, when exosomes isolated from the blood of tumor mice were solely administered (mExo only), the weight of lung and nodules metastasized to lung was significantly increased compared to other groups (FIGS. 14E and 14F). This was an unexpected result, suggesting that tumor metastasis was promoted when exosomes circulating in the blood of tumor mice were administered after separation outside the body.

On the other hand, as a result of measuring the weight of liver, there was no significant difference among all groups (FIG. 14G). Compared to phosphate buffer, the group treated with mEXO alone, and the doxorubicin-administered group, the survival rate was significantly extended in the groups treated with mEXO+ExoPep-KLA and ExoPep-KLA (FIG. 14H). The results suggest that the intravenously injected ExoPep-KLA peptide specifically bound to circulating exosomes in tumor mice, promoted internalization into tumor tissues and tumor cells, and subsequently, induced apoptosis of cancer cells. It also suggests that a similar anticancer therapeutic effect was derived even when blood-derived exosomes were administered while the exosomes are bound to ExoPep. After treatment, liver tissue was excised to check the accumulation of exosomes (mEXO-DiD labeled), but no accumulation was observed in all treatment groups (FIG. 14I).

<Example 15> Analysis of blood levels and function of liver and kidney after administration of ExoPep-KLA peptide in an A549 tumor mouse model

In order to investigate the systemic side effects due to the treatment of ExoPep-KLA peptide, mouse blood was collected after the treatment in Example 14, and blood levels and functional levels of liver and kidney were analyzed.

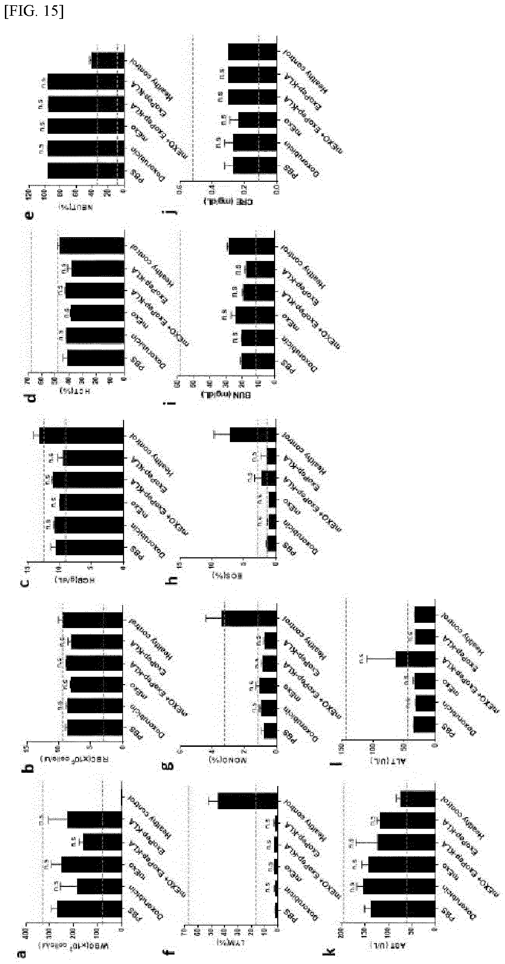

As a result, between the group treated with ExoPep-KLA or mEXO+ExoPep-KLA peptide and healthy mice, there was no significant difference beyond the normal range in blood levels including the white blood cell counts (FIG. 15). In addition, as a result of function tests for liver and kidney in the treatment group, no particular toxicity was shown (FIG. 15).

<Example 16> Inhibition of growth and metastasis of tumor by co-administration of doxorubicin and ExoPep-KLA peptide in an A549 tumor mouse model

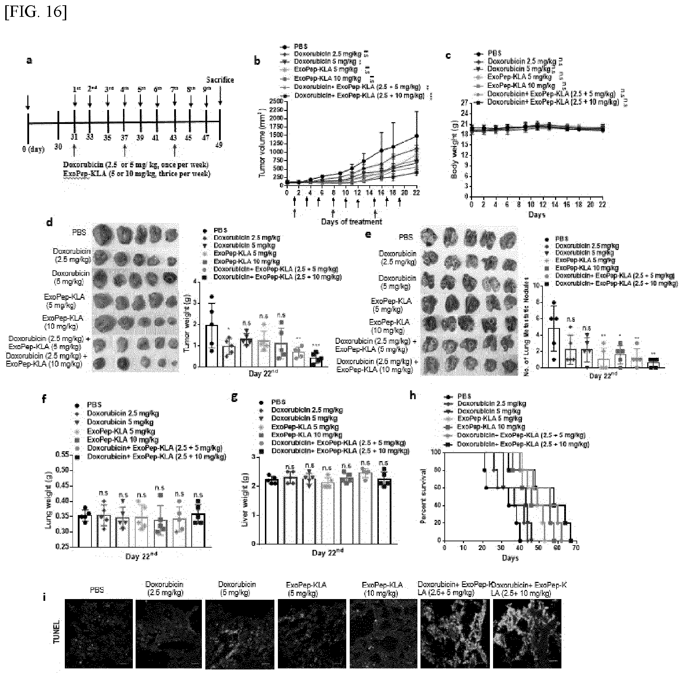

In the A549 tumor nude mouse model, ExoPep-KLA peptide was administered three times a week at the indicated time points, and doxorubicin was administered once a week for three weeks. The timing of single and co-administration was shown in the treatment protocol (FIG. 16A).

As a result, in the group solely treated with doxorubicin (2.5 mg/kg), ExoPep-KLA (5 mg/kg), and ExoPep-KLA (10 mg/kg), the size of tumor was slightly reduced compared to the group treated with the phosphate buffer. However, there was no significant difference (FIG. 16B). On the other hand, when doxorubicin (5 mg/kg), doxorubicin+ExoPep-KLA (2.5+5 mg/kg), and doxorubicin+ExoPep-KLA (2.5+10 mg/kg) were co-administered, tumors were more strongly inhibited. The body weight of the mice did not change during the treatment period despite the continuous administration of the exosome peptide and the combination with doxorubicin (FIG. 16C). In addition, the co-administration of doxorubicin+ExoPep-KLA (2.5+10 mg/kg) and doxorubicin+ExoPep-KLA (2.5+5 mg/kg) significantly decreased the weight of tumor. Such effect was most distinctive in the doxorubicin+ExoPep-KLA (2.5+10 mg/kg) combined group compared to other groups (FIG. 16D). In addition, compared to the buffer-treated group, metastatic nodules and microscopic nodules were significantly reduced in groups treated with doxorubicin (5 mg/kg), doxorubicin+ExoPep-KLA (2.5+5 mg/kg), and doxorubicin+ExoPep-KLA (2.5+10 mg/kg) (FIG. 16E).

In addition, as a result of measuring the weight of lung, there was no significant difference among all groups (FIG. 16F). No significant difference was observed among all groups in the result of measuring the weight of liver (FIG. 16G).

On the other hand, compared to the phosphate buffer and single treatment group, the survival rate was significantly increased in the group co-treated with doxorubicin+ExoPep-KLA (2.5+5 mg/kg) and doxorubicin+ExoPep-KLA (2.5+10 mg/kg) (FIG. 16H). In addition, after treatment, the tumor tissue was sectioned, and TUNEL staining (green) was observed in the tissue. As a result of immunohistochemical analysis, treatment of doxorubicin+ExoPep-KLA (2.5+5 mg/kg) and doxorubicin+ExoPep-KLA (2.5+10 mg/kg) significantly increased the proportion of TUNEL-positive apoptotic cells in tumor tissues. (FIG. 16I). The above results suggest that when ExoPep-KLA and doxorubicin were co-administered, the therapeutic effect of doxorubicin may be increased, and side effects thereof may be diminished through reduction of the dose later.

<Example 17> Analysis of blood levels and functional levels of liver and kidney after co-administration of ExoPep-KLA peptide and doxorubicin in an A549 tumor mouse model

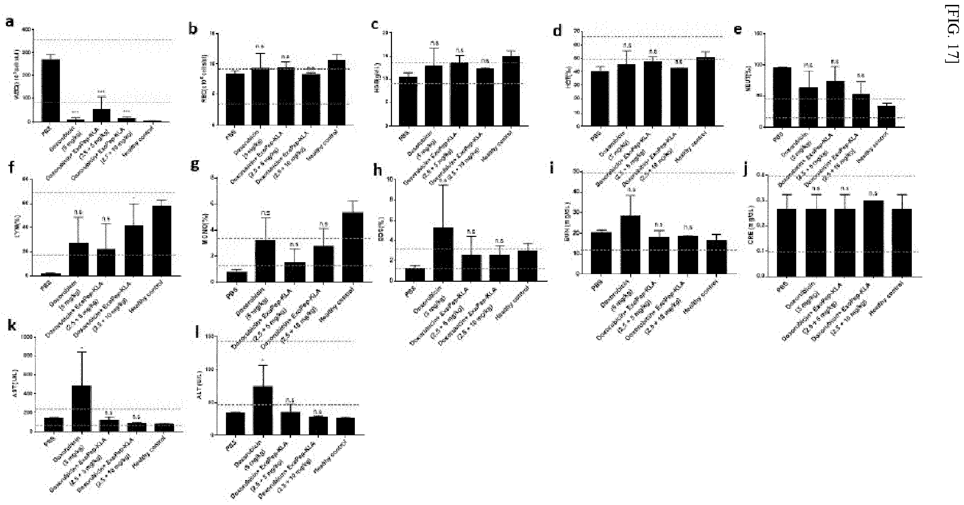

After the treatment in FIG. 16, blood was collected to analyze levels of blood and liver and kidney functions. As a result, there was no significant difference beyond the normal range in blood levels including white blood cell counts between the treated group and healthy mice, supporting that there were no systemic side effects due to co-treatment of ExoPep-KLA or doxorubicin+ExoPep-KLA (2.5+10 mg/kg). In addition, as a result of function tests for liver and kidney, compared to healthy mice, co-treatment of ExoPep-KLA or doxorubicin+ExoPep-KLA (2.5+10 mg/kg) did not show toxicity (FIG. 17).

<Example 18> Inhibition of growth and metastasis of tumor by administration of ExoPep-KLA peptide alone and co-administration of gemcitabine in a Panc-1 pancreatic cancer mouse model

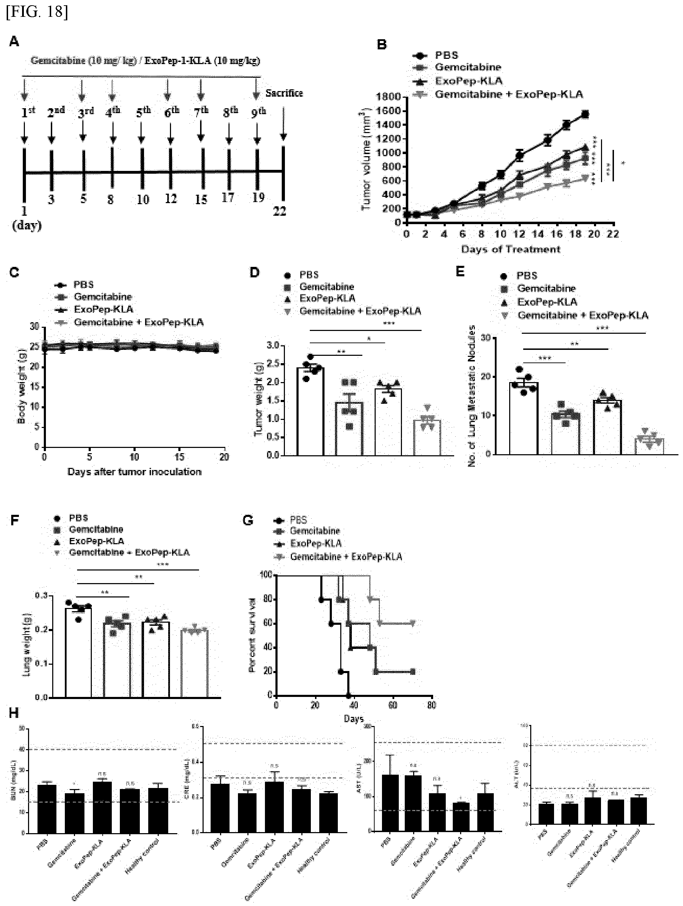

In the Panc-1 tumor model, anti-tumor effect for administration of ExoPep-KLA alone and co-administration with gemcitabine was identified (FIG. 18A). Systemic co-administration of ExoPep-KLA and gemcitabine significantly inhibited growth of tumor, and ExoPep-KLA administered group and PBS group did not significantly inhibit growth of tumor (FIG. 18B). In an attempt to identify the biological safety of the exosome peptide and the co-administered group, the inventors of the present disclosure observed a change in body weight during the administration period. No change was observed in the weight of the mouse during administration of the exosome peptide (FIG. 18C). Importantly, the group co-administered with ExoPep-KLA and gemcitabine decreased growth of primary tumor.

Compared to the group administered with ExoPep-KLA alone and the PBS group, it was observed that the weight of tumor decreased in the co-administered group (FIG. 18D). Compared to the PBS group, the group co-administered with ExoPep-KLA and gemcitabine barely showed metastatic or micronodules (FIG. 18E). In addition, the weight of lung was measured at the end of administration (FIG. 18F). On the other hand, the weight of primary tumor decreased in the co-administered group, and lifespan increased (FIG. 18G). No systemic side effects due to administration were observed in all groups (FIG. 18H).

As the specific parts of the present disclosure have been described in detail above, for those of ordinary skill in the art, it is clear that these specific descriptions are only preferred example embodiments, and the scope of the present disclosure is not limited thereby. Accordingly, it is intended that the substantial scope of the present disclosure may be defined by the appended claims and equivalents thereof.

## Claims

1. A peptide specifically binding to a cancer cell-derived exosome, comprising an amino acid sequence represented by SEQ ID NO: 1.

2. The peptide of claim 1, wherein the cancer cell is a lung cancer cell, a breast cancer cell, or a pancreatic cancer cell.

3. A polynucleotide encoding the peptide of claim 1.

4. A recombinant vector comprising the polynucleotide of claim 3.

5. A transformant transformed with the recombinant vector of claim 4.

6. A composition for diagnosing cancer, comprising the peptide of claim 1 as an active ingredient.

7. The composition of claim 6, wherein the peptide specifically binds to a cancer cell-derived exosome.

8. The composition of claim 6, wherein the cancer is one or more selected from the group consisting of lung cancer, breast cancer, pancreatic cancer, brain tumor, liver cancer, skin cancer, esophageal cancer, testicular cancer, kidney cancer, colorectal cancer, rectal cancer, stomach cancer, bladder cancer, ovarian cancer, bile duct cancer, gallbladder cancer, uterine cancer, cervical cancer, prostate cancer, head and neck cancer, and squamous cell carcinoma.

9. A composition for drug delivery, comprising the peptide of claim 1 as an active ingredient.

10. The composition of claim 9, wherein the drug is a peptide drug or an anticancer agent.

11. The composition of claim 10, wherein the peptide drug is a cytotoxic peptide having activity of inducing apoptosis or necrosis.

12. The composition of claim 10, wherein the anticancer agent is one or more selected from the group consisting of mertansine, doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, adriamycin, methotrexate, busulfan, chlorambucil, cyclophosphamide, melphalan, nitrogen mustard, and nitrosourea.

13. A fusion peptide in which an apoptosis-inducing peptide comprising an amino acid sequence represented by SEQ ID NO: 2 is bound to the peptide of claim 1.

14. A pharmaceutical composition for preventing or treating cancer, comprising the fusion peptide of claim 13 as an active ingredient.

15. A pharmaceutical composition for preventing or treating cancer, comprising the fusion peptide of claim 13 and an anticancer agent as active ingredients.

16. The pharmaceutical composition of claim 15, wherein the anticancer agent is one or more selected from the group consisting of mertansine, doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, adriamycin, methotrexate, busulfan, chlorambucil, cyclophosphamide, melphalan, nitrogen mustard, and nitrosourea.

17. The pharmaceutical composition of any one of claims 14 to 16, wherein the cancer is one or more selected from the group consisting of lung cancer, breast cancer, pancreatic cancer, brain tumor, liver cancer, skin cancer, esophageal cancer, testicular cancer, kidney cancer, colorectal cancer, rectal cancer, stomach cancer, bladder cancer, ovarian cancer, bile duct cancer, gallbladder cancer, uterine cancer, cervical cancer, prostate cancer, head and neck cancer, and squamous cell carcinoma.

18. A composition for imaging cancer cells, comprising the peptide of claim 1 as an active ingredient.

19. The composition of claim 18, wherein the peptide is labeled with any one selected from the group consisting of a chromogenic enzyme, a radioisotope, a chromophore, a luminescent material, a fluorescer, a magnetic resonance imaging material, superparamagnetic particles, and ultrasuper paramagnetic particles.

20. A composition for detecting cancer cell-derived exosomes, comprising the peptide of claim 1 as an active ingredient.

[FIG. 1]

**a**

Exosome (UC)

Exosome (ExoQ)

**c**

[FIG. 2]

[FIG. 3]

[FIG. 4]

[FIG. 5]

[FIG. 6]

[FIG. 7]

[FIG. 8]

[FIG. 9]

[FIG. 10]

[FIG. 11]

[FIG. 12]

[FIG. 13]

[FIG. 14]

[FIG. 15]

[FIG. 16]

[FIG. 17]

[FIG. 18]

**INTERNATIONAL SEARCH REPORT**

| International application No. |
| --- |
| **PCT/KR2021/010657** |

**A. CLASSIFICATION OF SUBJECT MATTER**

**C07K 14/00**(2006.01)i; **C07K 7/08**(2006.01)i; **G01N 33/574**(2006.01)i; **A61K 47/42**(2006.01)i; **A61K 31/704**(2006.01)i; **A61K 38/00**(2006.01)i; **A61K 51/08**(2006.01)i

According to International Patent Classification (IPC) or to both national classification and IPC

**B. FIELDS SEARCHED**

Minimum documentation searched (classification system followed by classification symbols)

C07K 14/00(2006.01); A61K 38/00(2006.01); A61K 39/00(2006.01); A61K 39/12(2006.01); C07K 14/705(2006.01); C07K 7/06(2006.01); C12N 5/078(2010.01); C12N 5/09(2010.01); C12N 5/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: CRKVAKG, ExoPep, 엑소좀(exosome), 항암(anti-cancer), 세포 내 유입(internalization)

**C. DOCUMENTS CONSIDERED TO BE RELEVANT**

| Category* | Citation of document, with indication, where appropriate, of the relevant passages | Relevant to claim No. |
| --- | --- | --- |
| A | CARNEY, R. P. et al. Targeting tumor-associated exosomes with integrin-binding peptides. Advanced biosystems. 2017, vol. 1, no. 5, HHS Public Access Author Manuscript Version internal inner pp. 1-28. See abstract; figure 1; and inner pages 4 and 10-11. | 1-20 |
| A | KR 10-2016-0130949 A (KOREA ADVANCED INSTITUTE OF SCIENCE AND TECHNOLOGY) 15 November 2016 (2016-11-15) See abstract; and claims 1-14. | 1-20 |
| A | US 2012-0321653 A1 (MAMOUN, R. Z. E. A.) 20 December 2012 (2012-12-20) See abstract; and claim 1. | 1-20 |
| A | US 2018-0015182 A1 (SANTA CLARA UNIVERSITY) 18 January 2018 (2018-01-18) See abstract; and claims 1-6. | 1-20 |
| A | KR 10-2019-0072466 A (KYUNGPOOK NATIONAL UNIVERSITY INDUSTRY-ACADEMIC COOPERATION FOUNDATION) 25 June 2019 (2019-06-25) See entire document. | 1-20 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 November 2021** | **15 November 2021** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2021/010657**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
Information on patent family members

| International application No. |
| --- |
| **PCT/KR2021/010657** |

| Patent document cited in search report | | | Publication date (day/month/year) | Patent family member(s) | | | Publication date (day/month/year) |
| --- | --- | --- | --- | --- | --- | --- | --- |
| KR | 10-2016-0130949 | A | 15 November 2016 | AU | 2016-258423 | A1 | 07 December 2017 |
| | | | | AU | 2017-335084 | A1 | 10 May 2018 |
| | | | | CA | 2983731 | A1 | 10 November 2016 |
| | | | | CA | 3002520 | A1 | 05 April 2018 |
| | | | | CN | 107980045 | A | 01 May 2018 |
| | | | | CN | 107980045 | B | 25 May 2021 |
| | | | | CN | 108473973 | A | 31 August 2018 |
| | | | | EP | 3293265 | A1 | 14 March 2018 |
| | | | | EP | 3356522 | A1 | 08 August 2018 |
| | | | | JP | 2018-515092 | A | 14 June 2018 |
| | | | | JP | 2019-528674 | A | 17 October 2019 |
| | | | | JP | 2021-118725 | A | 12 August 2021 |
| | | | | JP | 6878305 | B2 | 26 May 2021 |
| | | | | KR | 10-1877010 | B1 | 09 August 2018 |
| | | | | KR | 10-1900465 | B1 | 19 September 2018 |
| | | | | KR | 10-1912310 | B1 | 26 October 2018 |
| | | | | KR | 10-1912313 | B1 | 26 October 2018 |
| | | | | KR | 10-1912315 | B1 | 26 October 2018 |
| | | | | KR | 10-2016-0130937 | A | 15 November 2016 |
| | | | | KR | 10-2018-0036134 | A | 09 April 2018 |
| | | | | KR | 10-2018-0036381 | A | 09 April 2018 |
| | | | | KR | 10-2018-0036402 | A | 09 April 2018 |
| | | | | KR | 10-2018-0037399 | A | 12 April 2018 |
| | | | | KR | 10-2018-0040834 | A | 23 April 2018 |
| | | | | KR | 10-2018-0092874 | A | 20 August 2018 |
| | | | | KR | 10-2120921 | B1 | 10 June 2020 |
| | | | | US | 10702581 | B2 | 07 July 2020 |
| | | | | US | 2018-0117117 | A1 | 03 May 2018 |
| | | | | US | 2020-0289614 | A1 | 17 September 2020 |
| | | | | WO | 2016-178532 | A1 | 10 November 2016 |
| | | | | WO | 2018-062973 | A1 | 05 April 2018 |
| US | 2012-0321653 | A1 | 20 December 2012 | CA | 2775151 | A1 | 31 March 2011 |
| | | | | CA | 2775151 | C | 22 January 2019 |
| | | | | EP | 2480672 | A1 | 01 August 2012 |
| | | | | EP | 2480672 | B1 | 28 December 2016 |
| | | | | EP | 2480672 | B8 | 22 March 2017 |
| | | | | FR | 2950350 | A1 | 25 March 2011 |
| | | | | JP | 2013-505713 | A | 21 February 2013 |
| | | | | JP | 5932647 | B2 | 08 June 2016 |
| | | | | US | 9611481 | B2 | 04 April 2017 |
| | | | | WO | 2011-036416 | A1 | 31 March 2011 |
| US | 2018-0015182 | A1 | 18 January 2018 | US | 10617768 | B2 | 14 April 2020 |
| | | | | US | 10758486 | B2 | 01 September 2020 |
| | | | | US | 2019-0015333 | A1 | 17 January 2019 |
| | | | | US | 2020-0306297 | A1 | 01 October 2020 |
| KR | 10-2019-0072466 | A | 25 June 2019 | EP | 3725799 | A1 | 21 October 2020 |
| | | | | KR | 10-2150419 | B1 | 01 September 2020 |
| | | | | US | 2021-0163534 | A1 | 03 June 2021 |
| | | | | WO | 2019-117690 | A1 | 20 June 2019 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Proteins. **CREIGHTON.** Structures and Molecular Principles. W. H. Freeman and Co, 1983
- Chemical Approaches to the Synthesis of Peptides and Proteins. CRC Press, 1997
- A Practical Approach. IRL Press, 1989
- **WU et al.** *J. Bio. Chem.,* 1992, vol. 267, 963-967
- **WU ; WU.** *J. Bio. Chem.,* 1988, vol. 263, 14621-14624